# EUROPÄISCHE PATENTSCHRIFT

(11) **EP 1 536 838 B1**
(45) Veröffentlichungstag und Bekanntmachung des Hinweises auf die Patenterteilung: **31.01.2007**
(21) Anmeldenummer: 03798884.7
(22) Anmeldetag: 02.09.2003
(51) Int. Cl.: A61K 47/48, A61K 39/35

(54) **Hyposensibilisierung durch Hydoxyalkylstärke-Allergen-Konjugate**
Hyposensibilisation by hydroxyalkyl starch allergen conjugates
Hyposensibilisation par conjugés d'amidon hydroxyalkyle et allergène

(30) Priorität: 11.09.2002 DE 10242076
(43) Veröffentlichungstag der Anmeldung: 08.06.2005
(73) Patentinhaber: Fresenius Kabi Deutschland GmbH, 61352 Bad Homburg v.d.H. (DE)
(72) Erfinder: EICHNER, Wolfram, 35510 Butzbach (DE); DORMANN, Dirk, 55246 Mainz-Kostheim (DE)
(74) Vertreter: Wichmann, Hendrik
(86) Internationale Anmeldenummer: PCT/EP2003/009750
(87) Internationale Veröffentlichungsnummer: WO 2004/030701

(56) Entgegenhaltungen:
- WO-A-97/30148
- WO-A-02/080979
- WO-A-03/074087
- US-A- 4 261 973
- SCHAEFER T ET AL: "TWO-YEAR DOUBLE-BLIND TRIAL OF A MONOMETHOXY POLYETHYLENE GLYCOL MPEG MODIFIED GRASS POLLEN EXTRACT AT DIFFERENT DOSE LEVELS" ANNALS OF ALLERGY, Bd. 68, Nr. 4, 1992, Seiten 334-339, XP009022545 ISSN: 0003-4738
- DREBORG S ET AL: "IMMUNOTHERAPY WITH MONOMETHOXYPOLYETHYLENE GLYCOL MODIFIED ALLERGENS" CRITICAL REVIEWS IN THERAPEUTIC DRUG CARRIER SYSTEMS, Bd. 6, Nr. 4, 1990, Seiten 315-365, XP009022546 ISSN: 0743-4863

## Beschreibung

Die vorliegende Erfindung betrifft Verbindungen, die ein Konjugat aus einer Hydroxyalkylstärke (HAS) und einem Allergen umfassen, wobei die HAS entweder unmittelbar oder über einen Linker kovalent an das Allergen gebunden ist. Die Erfindung betrifft genauer die Verwendung entsprechender Konjugate zur Herstellung eines Arzneimittel, zur Hyposensibilisierung.

### TECHNISCHER HINTERGRUND

Überschiessende, spezifische Reaktionen des Immunsystems gegen exogene Substanzen werden heute unter dem Begriff der Allergie zusammengefasst. Nach der Einteilung von Coombus und Gell lassen sich allergische Reaktionen in die Typen I bis IV untergliedern, die u.a. auf Basis der in die Reaktion involvierten Antikörperklassen, der erkannten Antigene sowie der induzierten Effektormechanismen differenziert werden können.

Als Allergene werden demgemäß Verbindungen bezeichnet, die eine allergische Immunreaktion, im engeren Sinne eine allergische Immunreaktion vom Soforttyp (Typ I), an Haut und Schleimhaut auslösen können. In der Regel handelt es sich bei den Ällergenen um Polypeptide oder Proteine mit einem Molekulargewicht von etwa 5.000 bis etwa 80.000 Da. Die Polypeptide können pflanzlichen, tierischen oder mikrobiologischen Ursprungs sein. Die Polypeptide können ferner als Bestandteile des Hausstaubs vorliegen.

Allergene induzieren IgE Antikörper, welche mit ihrem konstanten Teil an die Oberfläche von Mastzellen binden und dadurch eine Degranulation der Mastzellen bewirken. Die von Mastzellen freigesetzten Stoffe (Histamine, proteolytische Enzyme und Entzündungsmediatoren) verursachen unmittelbar und mittelbar die Symptome einer Allergie, üblicherweise Rhinitis, Konjunktivitis und/oder bronchiales Asthma.

IgE-vermittelte Soforttypallergien (Typ I) sind die mit Abstand am stärksten vorherrschende allergische Reaktionsform. Bis zu 20% der Menschen in Industrieländern leiden an Typ I-allergischen Symptomen. Allergiker werden derzeit neben der medikamentösen Therapie durch spezifische Immuntherapie, die sogenannten Hyposensibilisierungen, behandelt (Kleine-Tebbe et al., Pneumologie, Vol. 5 (2001), 438-444).

Bei der klassischen Hyposensibilisierung wird ein spezifischer Allergenextrakt subkutan in ansteigenden Quantitäten verabreicht, bis eine individuelle Erhaltungsdosis erreicht wird. Bei Fortführung der Behandlung wird diese Dosis wiederholt verabreicht, wobei verschiedene Behandlungsprotokolle zum Einsatz gelangen (Klimek et al., Allergologie und Umweltmedizin, Schattauer Verlag, S. 158 ff.).

Der Therapieerfolg scheint dabei in engem Zusammenhang mit den während der Erhaltungsphase eingesetzten Allergenquantitäten zu stehen. Bei Erhöhung der verabreichten Allergenquantitäten steigt jedoch grundsätzlich auch das Risiko einer IgE-vermittelten Reaktion des allergischen Patienten an. Der Einsatz der Therapie wird mit anderen Worten auch durch die allergische Reaktion des Patienten und das damit für den Patienten einhergehende Risiko eines anaphylaktischen Schocks eingeschränkt.

Ein Erfolg der Therapie wird in einer Verminderung der allergischen Symptome gesehen, die zu einem individuellen Rückgang des Medikamentenbedarfs bzw. einem Anstieg der Toleranz gegenüber dem Allergen führt.

Es wurde bereits vorgeschlagen, einzelne allergene Polypeptide durch rekombinante Expression zu erzeugen und für eine Hyposensibilisierung zu verwenden (DE 100 41 541).

Um Allergene mit verringerten IgE-Bindungseigenschaften zu gewinnen, wurden diese mit Polyethylenglykol (PEG) modifiziert und für die Hyposensibilisierung verwendet. Eine Vielzahl von Veröffentlichungen beschreibt dementsprechend die Herstellung von PEG-Allergen-Konjugaten, welche durch kovalente Bindung eines Allergens an Polyethylenglykol erzeugt wurden. Mosbech et al. (Allergy, 1990, Vol. 45(2):130-141) berichten beispielsweise die Behandlung von erwachsenen asthmatischen Allergikern mit PEG-Hausstaub-Konjugaten und die immunologische Reaktion nach der Behandlung. Die Autoren fanden eine klinische Verbesserung der Wirkung, sofern die Dosierung des Allergens ausreichte, um die Menge des spezifischen IgE zu reduzieren und/oder IgG, insbesondere IgG4, Reaktionen zu induzieren.

Ähnlich berichten Schafer et al., (Ann. Allergie, 1992, Vol. 68(4):334-339) über eine Studie, in der eine allergene Zusammensetzung aus einem PEG-modifiziertem Graspollenmix für die Hyposensibilisierung von Erwachsenen verwendet wurde. Die Ergebnisse wurden mit denen verglichen, die durch Hyposensibilisierung unter Verwendung des teilweise gereinigten Graspollenmix erhalten wurden. Die Behandlung erfolgte im Rahmen einer Doppelblindstudie. Die Frequenz und das Ausmaß der Nebenwirkungen konnte durch PEG-Modifizierung um etwa 50% verringert werden. Eine signifikante Verbesserung der Überempfindlichkeit wurde in beiden Behandlungsgruppen festgestellt.

PEG-Konjugate weisen jedoch keine in der Natur vorkommende Struktur auf, für die *in vivo* Abbauwege beschrieben wurden.

Neben den PEG-Konjugaten sind auch andere Allergen-Derivate hergestellt und untersucht worden. So sind Dextran-modifizierte Allergene bekannt, die durch Konjugation mit Carboxymethyldextran erzeugt werden. Einige Studien mit beta-Laktoglobulin haben gezeigt, dass die Antikörperreaktion gegenüber Dextran-Konjugaten im Vergleich mit nicht-modifizierten Verbindungen erheblich abgeschwächt ist (Kobayashi et al., J Agric Food Chem 2001 Feb; 49(2): 823-31; Hattori et al., Bioconjug Chem 2000 Jan-Feb; 11(1): 84-93).

Ferner wurden kreuzvernetzte, hochmolekulare Allergene erzeugt, sogenannte Allergoide. Diese Produkte konnten beispielsweise mittels Formaldehyd- oder Glutaraldehyd-Modifikation von Allergenen gewonnen werden. Entsprechende Produkte können von Allergopharma, Joachim Ganser KG, 21462 Reinbek; HAL Allergie GmbH, 40554 Düsseldorf; und SmithKline Beecham Pharma GmbH, Benckard, 80716 München, erhalten werden.

US 4,261,973 beschreibt ein Verfahren zur Unterdrückung der Produktion von Antikörpern gegen ein Allergen, wobei ein Allgergen-Polymer-Konjugat verabreicht wird, bei das Allergen an wasserlösliche Polymere gekoppelt ist.

WO 97/30148 betrifft Polypeptidkonjugate mit erniedrigter Allergenität umfassend ein polymeres Trägermolekül, an das ein oder mehrere Polypeptidmoleküle gekoppelt sind. Darüber hinaus offenbart die Anmeldung ein Verfahren zur Herstellung der Polypeptidkonjugate mit erniedrigter Allergenität, Zusammensetzungen enthaltend diese und die Verwendung in einer Reihe gewerblicher Anwendungen wie z.B. in Pflegeprodukten und Reinigungsmitteln.

Einen umfassenden Überblick über die Bandbreite verschiedener Verfahren zur Herstellung von Biokonjugaten im Allgemeinen gibt G.T. : Hermanson (Bioconjugate Techniques, Academic Press, San Diego 1996). Die Verknüpfung von Oligo- und Polysacchariden mit Proteinen erfolgt dabei zumeist über Lysin- (-NH₂) oder Cystein-(-SH) Seitenketten und seltener über Asparagin- oder Glutaminsäure- (-COOH) oder auch Tyrosin-(Aryl-OH) Seitenketten.

Stärkederivate sind bisher jedoch für die Modifizierung von Allergenen nicht verwendet worden.

Hydroxyethylstärke beispielsweise ist ein substituiertes Derivat des in Maisstärke zu 95 % vorkommenden Kohlenhydrat-Polymers Amylopektin. HES weist vorteilhafte rheologische Eigenschaften auf und wird zur Zeit als Volumenersatzmittel und zur Hämodilutionstherapie klinisch eingesetzt (Sommermeyer et al., Krankenhauspharmazie, Vol. 8(8), (1987), S.271-278; und Weidler et al., Arzneim.-Forschung/ Drug Res., 41, (1991) 494-498).

Amylopektin besteht aus Glucoseeinheiten, wobei in den Hauptketten α-1,4-glykosidische Bindungen vorliegen, an den Verzweigungsstellen jedoch α-1,6-glykosidische Bindungen. Die physikalisch-chemischen Eigenschaften dieses Moleküls werden im Wesentlichen durch die Art der glykosidischen Bindungen bestimmt. Aufgrund der abgeknickten α-1,4-glykosidischen Bindung entstehen helikale Strukturen mit etwa 6 Glucose-Monomeren pro Windung.

Die physikalisch-chemischen als auch die biochemischen Eigenschaften des HES-Polymers können durch Substitution verändert werden. Die Einführung einer Hydroxyethylgruppe kann durch alkalische Hydroxyethylierung erreicht werden. Durch die Reaktionsbedingungen kann die unterschiedliche Reaktivität der jeweiligen Hydroxylgruppe im unsubstituierten Glucosemonomer gegenüber der Hydroxyethylierung ausgenutzt werden, wodurch eine Einflussnahme auf das Substitutionsmuster möglich ist.

Daher wird HES im Wesentlichen über die Molekulargewichtsverteilung und den Substitutionsgrad gekennzeichnet. Der Substitutionsgrad kann dabei als DS ("degree of substitution"), welcher auf den Anteil der substituierten Glucosemonomere aller Glucoseeinheiten Bezug nimmt, oder als MS ("molar substitution") beschrieben werden, womit die Anzahl von Hydroxyethylgruppen pro Glucoseeinheit bezeichnet wird.

HES-Lösungen liegen als polydisperse Zusammensetzungen vor, in denen sich die einzelnen Moleküle hinsichtlich des Polymerisationsgrades, der Anzahl und Anordnung der Verzweigungsstellen sowie ihres Substitutionsmusters voneinander unterscheiden. HES ist somit ein Gemisch von Verbindungen mit unterschiedlichem Molekulargewicht. Dementsprechend wird eine bestimmte HES-Lösung durch ein durchschnittliches Molekulargewicht anhand statistischer Größen bestimmt. Dabei wird Mₙ als einfaches arithmetisches Mittel in Abhängigkeit von der Anzahl der Moleküle errechnet (Zahlenmittel), während M_{w}, das Gewichtsmittel, die masseabhängige Messgröße darstellt.

Der vorliegenden Erfindung liegt somit die Aufgabe zugrunde, verbesserte Allergen-Derivate zur Verfügung zu stellen, insbesondere Allergen-Derivate, die einen Depot-Effekt erzielen und daher weniger oft verabreicht werden müssen, die zur Herstellung von Arzneimitteln zur Hyposensibilisierung verwendet werden.

Diese Aufgabe wurde nunmehr durch die Verwendung von Konjugaten aus Hydroxyalkylstärke (HAS) und Allergen zur Herstellung eines Arzneimittels zur Hyposensibilisierung gelöst, bei denen mindestens eine Hydroxyalkylstärke kovalent an das Allergen gekoppelt ist.

Erfindungsgemäß wurde demgemäß überraschenderweise festgestellt, dass die HAS-Allergen-Konjugate in besonders vorteilhafter Weise für die spezifische Immuntherapie verwendet werden können. Die Sicherheit der Hyposensibilisierung wird durch die Verwendung der erfindungsgemäßen Konjugate erhöht. Gleichzeitig weisen die erfindungsgemäßen Konjugate eine höhere *in vivo* Halbwertszeit auf, womit durch die Konjugierung mit HAs ein Depoteffekt erzielt wird, der die klinische Wirksamkeit positiv beeinflusst. Insbesondere gegenüber wässrigen Allergenextrakten weist der Depoteffekt der erfindungsgemäßen Konjugate den Vorteil auf, dass diese weniger häufig verabreicht werden müssen, um eine therapeutische Wirkung zu erzielen.

Im Vergleich zu nicht-modifizierten Allergenen können die beschriebenen HAS-Allergen-Konjugate so hergestellt werden, dass sie eine verringerte Bindung an Allergen-spezifisches IgE aufweisen. Die HAS-Allergen-Konjugate können gemäß einer besonders bevorzugten Ausführungsform nur eine sehr geringe oder gar keine spezifische Bindung an Allergen-spezifisches IgE aufweisen. Die erfindungsgemäßen Konjugate können somit in höherer Dosierung appliziert werden, was wiederum die Wahrscheinlichkeit einer erfolgreichen Hyposensibilisierung erhöht.

Gegenüber vernetzten Allergoiden weisen die HAS-Allergen-Konjugate den Vorteil auf, dass sie ein dem natürlichen Allergen vergleichbares Epitop-Profil liefern können. Die Effizienz der Immuntherapie kann somit erhöht werden. Demgegenüber führt die Polymerisation von Allergenen mittels Formaldehyd oder Glutaraldehyd zu schlecht definierten hochmolekularen Verbindungen (Crit Rev Ther Drug Carrier Syst 1990; 6(4): 315-65), die nicht-natürliche Epitope erzeugen können, so dass deren Wirkung im Einzelfall untersucht werden müßte.

In dem Konjugat ist mindestens eine Hydroxyalkylstärke an ein Allergen gekoppelt. Vom Umfang der Erfindung umfaßt ist natürlich auch die Verwendung von Kopplungsprodukten, die mehrere Hydroxyalkylstärke-Moleküle und ein Allergen-Molekül oder mehrere Allergen-Moleküle und ein Hydroxyalkylstärke-Molekül aufweisen.

Die Hydroxyalkylstärke kann unmittelbar an das Allergen oder über einen Linker an das Allergen gekoppelt in dem Konjugat vorliegen. Ferner kann die Hydroxyalkylstärke an die Polypeptidkette oder an eine oder mehrere der Saccharidketten eines allergenen Glykoproteins gekoppelt sein.

### HYDROXYALKYLSTÄRKE (HAS).

Im Rahmen der vorliegenden Erfindung wird der Begriff "Hydroxyalkylstärke" dazu verwendet, um Stärkederivate zu bezeichnen, die mit einer Hydroxyalkylgruppe substituiert wurden. Vorzugsweise umfaßt die Hydroxyalkylgruppe 2 bis 4 C-Atomen. Die als "Hydroxyalkylstärke" bezeichnete Gruppe umfaßt somit -vorzugsweise Hydroxyethylstärke, Hydroxypropylstärke und Hydroxybutylstärke. Die Verwendung von Hydroxyethylstärke (HES) als Kopplungspartner ist für alle Ausführungsformen der Erfindung besonders bevorzugt.

Erfindungsgemäß ist es bevorzugt, dass die Hydroxyethylstärke, die für die Verwendung der Konjugate eingesetzt wird, ein mittleres Molekulargewicht (Gewichtsmittel) von 1-300 kDa aufweist, wobei ein mittleres Molekulargewicht von 5 bis 200 kDa besonders bevorzugt ist. Hydroxyethylstärke kann ferner einen molaren Substitutionsgrad von 0,1-0,8 und ein Verhältnis von C₂:C₆-Substitution im Bereich von 2-20, jeweils bezogen auf die Hydroxyethylgruppen, aufweisen.

### ALLERGENE

Als Allergene werden im Rahmen der vorliegenden Erfindung in erster Linie Verbindungen bezeichnet, die allergische Immunreaktionen, im engeren sinne IgE-vermittelte Überempfindlichkeitsreaktionen (Typ I) auslösen können. Ferner umfaßt sind aus der Sequenz eines Allergens abgeleitete Peptide wie beispielsweise aus enzymatischen Spaltungen resultierende Spaltprodukte. Entsprechende Allergene werden für die spezifische Immuntherapie eingesetzt und sind kommerziell erhältlich.

Allergene können aus natürlichen Quellen isoliert werden. So werden z.B. im Fall der Pollenallergene Allergenextrakte aus den jeweiligen Pollen gewonnen. Ferner können die Allergene beispielsweise rekombinant hergestellt werden.

Vorzugsweise handelt es sich bei den Allergenen um Verbindungen, die aus der Gruppe bestehend aus Polypeptiden, Proteinen, und Glykoproteinen ausgewählt wurden.

### HERSTELLUNGSVERFAHREN

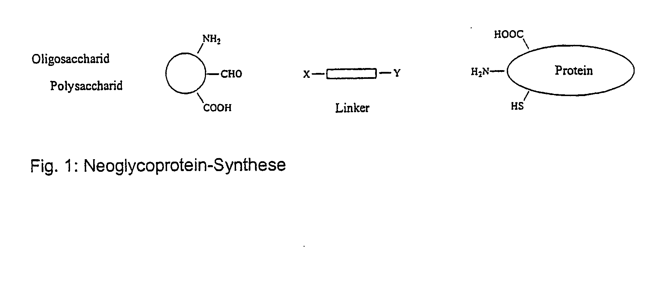

Gemäß eines Aspektes betrifft die Erfindung die Verwendung von HAS-Allergen-Konjugaten, bei denen man HAS entweder unmittelbar oder über einen Linker kovalent an das Allergen koppelt. Die Kopplung kann dabei auf verschiedenen Wegen erfolgen. Eine allgemeine Struktur für eine Neoglycoprotein-Synthese unter Verwendung eines Linkers wird in Fig. 1 gezeigt.

Gemäß einer Ausführungsform betrifft die vorliegende Erfindung die Verwendung von HAS-Allergen-Konjugaten, in denen HES an eine ε-NH₂-Gruppe, an eine α-NH₂-Gruppe, an eine SH-Gruppe, an eine COOH-Gruppe oder an eine -C(NH₂)₂-Gruppe eines Allergens gebunden wird.

Die Erfindung umfaßt ferner Verwendungen, bei denen man HES mittels reduktiver Aminierung an die ε-NH2-Gruppe eines Proteins koppelt. Alternativ dazu betrifft die Erfindung Verwendungen, bei denen das Allergen an die reduzierenden Endgruppen der Hydroxyethylstärke gekoppelt wird.

Gemäß einer weiteren Ausführungsform betrifft die Erfindung Verwendungen, bei denen für der Kopplung an das Allergen eine aktive Gruppe in die HAS eingeführt wird. Die aktive Gruppe kann beispielsweise eine Aldehyd-, Thiol- oder eine AminoFunktion sein.

Das Allergen und das Oligo- oder Polysaccharid können entweder direkt oder unter Verwendung eines Linkers aneinander gekoppelt werden. Als Linker kann ein beliebiges Vernetzungsmittel eingesetzt werden. Der Linker kann beispielsweise ein bifunktioneller Linker oder ein homo- oder heterobifunktioneller Cross-Linker sein.

Zahlreiche Vernetzungsmittel, wie beispielsweise SMCC (Succinimidyl-4-(N-maleimido-methyl)cyclohexan-1-carboxylat), sind kommerziell erhältlich und dem Fachmann geläufig (vgl. alphabetische Liste der "Cross-linking Reagents" im Produktkatalog der Firma Perbio und www.piercenet.com) und können im Rahmen der vorliegenden Erfindung zum Einsatz gelangen.

Nachfolgend werden einige Verfahren zur Synthese von HAS-Allergen-Konjugaten allgemein dargestellt. Der auf dem Gebiet der Biokonjugate tätige Durchschnittsfachmann wird keine Probleme haben, aus den beschriebenen Verfahren solche auszuwählen, die im Hinblick auf die zu lösenden Aufgaben (gewähltes Allergen, gewähltes HAS, etc.) besonders geeignet sind.

### Direkte Kopplung unmodifizierter HAS an allergene Proteine durch reduktive Aminierung:

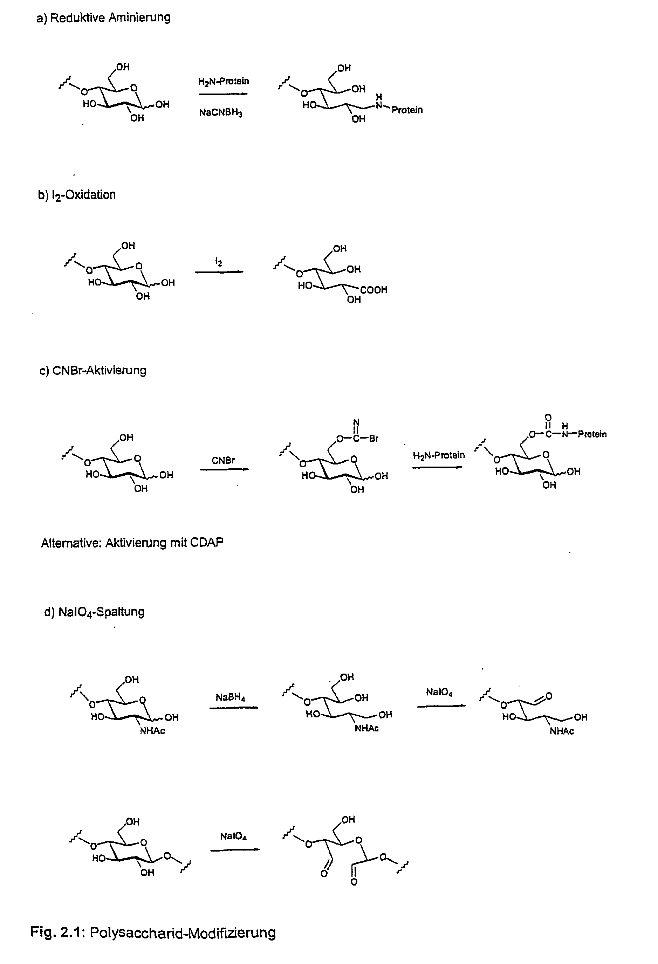

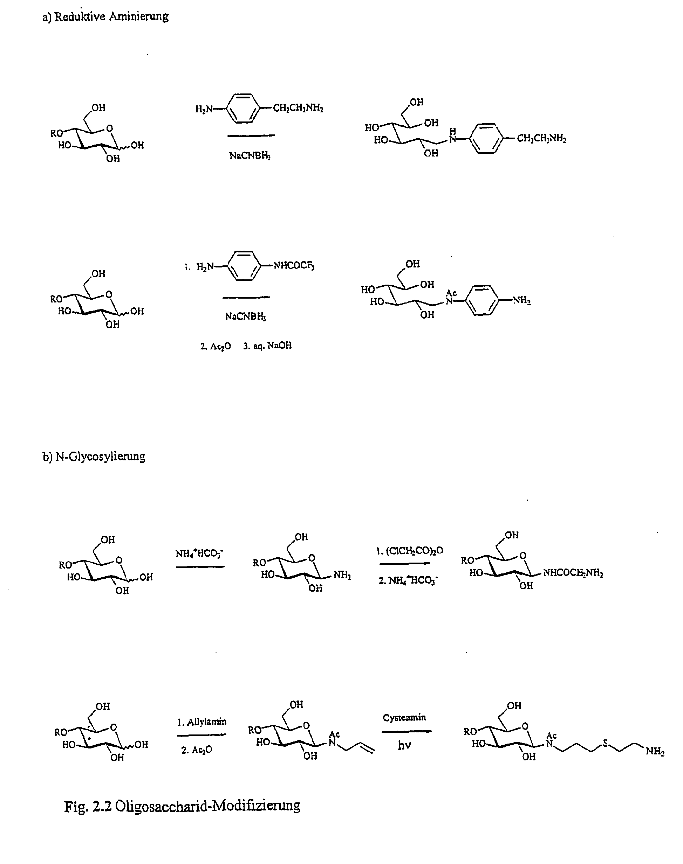

Ein einfaches und schonendes Verfahren, das ohne eine Modifizierung der HAS durchgeführt werden kann, stellt die direkte Kopplung der HAS an die ε-Aminogruppen des allergenen Proteins über eine reduktive Aminierung in Gegenwart von NaCN/BH₃ dar (G.R. Gray, Arch. Biochem. Biophys. 1974, 163, 426-28) (Fig. 2.1a).

Als Reduktionsmittel können auch pyridin-Boran und andere Amino-Boran Komplexe eingesetzt werden, die stabiler und leichter zu handhaben sind (J.C. Cabacungan et al., Anal. Biochem. 1982, 124, 272-78). Im Gegensatz zu einer Acylierung bleibt die modifizierte Aminogruppe des Proteins unter physiologischen Bedingungen weiterhin positiv geladen. Daher sind die Auswirkungen auf die Tertiärstruktur des Proteins bei der reduktiven Aminierung geringer. Bei diesem Verfahren geht jedoch die Ringstruktur des reduzierenden Zuckers verloren.

### Verfahren zur Kopplung von modifizierten HAS:

### Oxidation des reduzierenden Endes zu Aldonsäuren

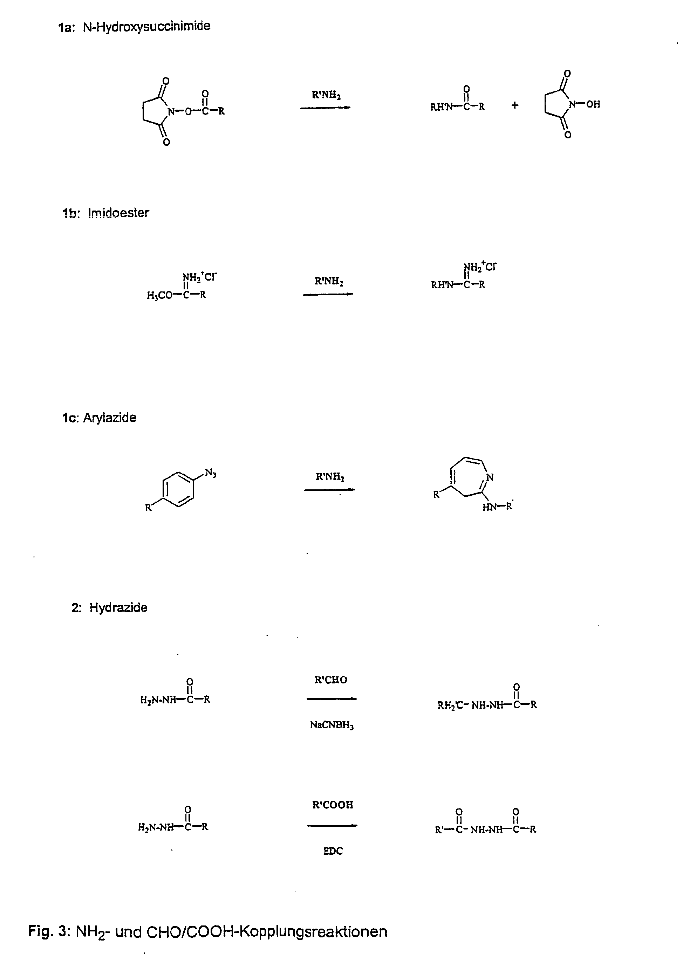

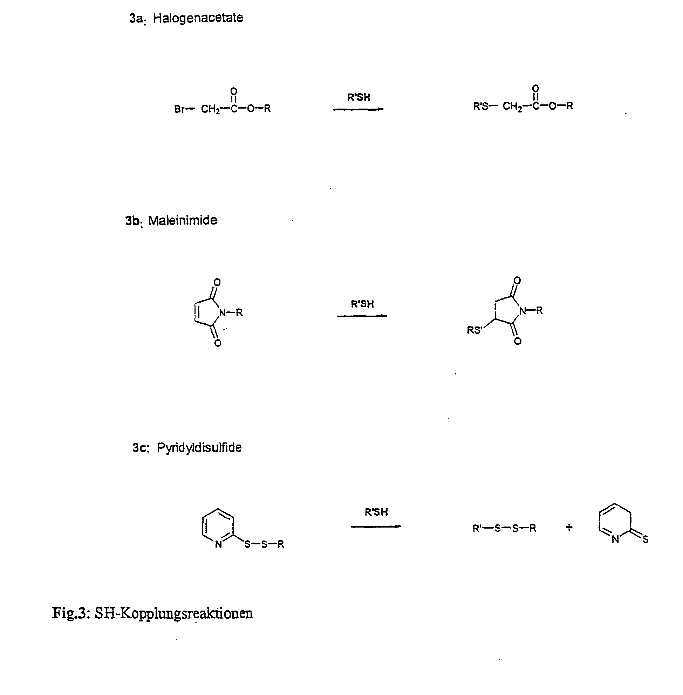

Bei der selten genutzten Oxidation mit Jod (oder Brom) zur entsprechenden Aldonsäure (G. Ashwell, Methods Enzymol. 1972, 28, 219-22) geht die Ringstruktur des reduzierenden Zuckers verloren (Fig. 2.1b), zudem ist eine sorgfältige Reaktionskontrolle nötig, um unspezifische Oxidation zu vermeiden. Die gebildete Carbonsäurefunktion kann in Gegenwart von EDC (1-Ethyl-3-(3-dimethylaminopropyl)carbodiimid) (J. Lönngren, I.J. Goldstein, Methods Enzymol. 1994, 247, 116-118) mit den ε-Aminogruppen der Lysinseitenketten des allergenen Proteins oder über einen Hydrazid-Linker. (-siehe Fig. 3) gekoppelt werden. Analog können auch die in den Polysaccharid-Strukturen vorliegenden Carboxyl-Gruppen von z.B. Mannuron-, Glucuron- oder Sialinsäuren zur Kopplung verwendet werden.

Gemäß einer besonders bevorzugten Ausführungsform werden Verbindungen für die erfindungsgemäße Verwendung bereitgestellt, die aus einem HES-Allergen-Konjugat bestehen, in der das Allergen spezifisch an die reduzierenden Endgruppen der Hydroxyethylstärke gebunden ist. Dafür kann man die reduzierenden Endgruppen zuvor selektiv oxidieren, beispielsweise nach dem in Hashimoto et al. (Kunststoffe, Kautschuk, Fasern, Vol. 9, (1992) S. 1271-1279) beschriebenen Verfahren zur Oxidation der reduzierenden Aldehyd-Endgruppe eines Saccharids.

### Aktivierung der Hydroxyfunktion der HAS

Eine der gebräuchlichsten Methoden für eine unspezifische Aktivierung von Polysacchariden stellt die Umsetzung mit Bromcyan (CNBr) dar (C. Chu et al., Infect. Immun. 1983, 40, 245-56) (Fig. 2.1c). Die aktivierten Hydroxygruppen acylieren.Lysin-, Cystein- und Histidin-Seitenketten des Proteins. Dieses Kopplungsverfahren kann jedoch Nachteile aufweisen, welche auf den hohen pH-Wert sowie auf die Toxizität und schlechte Handhabbarkeit zurückzuführen sind.

Eine Alternative zu CNBr bietet CDAP (1-Cyano-4-dimethylaminopyridiniumtetrafluorborat) (A. Lees et al., Vaccine 1996, 14, 190-98; D.E. Shafer et al., Vaccine 2000, 18, 1273-81) mit erhöhter Reaktivität der Cyano-Gruppe, das eine Reaktion unter sehr viel milderen Bedingungen ermöglicht.

Generell können die unspezifischen Aktivierungen von Polysacchariden zu einer mehrfachen Substitution und damit auch zu Kreuzvernetzungen zwischen Polysaccharid und Protein führen. Durch geeignete Wahl der Reaktionsbedingungen läßt sich dies jedoch weitgehend unterbinden.

### Einführung von Aldehyden

Auch in nicht reduzierenden Polysacchariden können Aldehyd-Funktionen durch die Spaltung vicinaler Hydroxygruppen mit NaIO₄ eingeführt werden (J.M. Bobbit, Ad. Carbohydr. Chem. 1956, 11, 1-41) (Fig. 2.1d), wobei über die Konzentration der Natriumperiodat-Lösung eine ausreichende Selektivität erreicht werden kann. Besonders leicht zu oxidieren ist Sialinsäure (S.M. Chamov et at., Biol. Chem 1992, 267, 15916-22).

Eine Erhöhung der Reaktionsgeschwindigkeit bei der direkten reduktiven Aminierung mit reduzierenden Polysacchariden kann durch die Einführung von Aldehydgruppen erreicht werden, die nicht zu Halbacetalen cyclisieren. Dies kann durch eine Reduktion des reduzierenden Endes zum Zuckeralkohol und einer anschließenden selektiven Oxidation der vicinalen Diole im geöffneten Zuckeralkohol erreicht werden (Y.C. Lee, R.T. Lee, Neoglycoproteins: Preparation and Application, Academic Press, San Diego 1994) (Fig. 2.1d).

Neben der direkten Kopplung der Aldehyd-modifizierten Polysaccharide mit Aminofunktionen des Proteins durch reduktive Aminierung ist auf diesem Weg auch eine Modifizierung des Polysaccharids mit bifunktionellen Hydrazid-Linkern möglich (siehe unten).

### Einführung von Amino-Funktionen

Gegenüber den Polysacchariden ergeben sich bei Oligosacchariden (mit bis zu 20 Kohlenhydrat-Monomeren) aufgrund der etwas höheren Reaktivität bessere Möglichkeiten, den reduzierenden Zucker über eine reduktive Aminierung zu Glycaminen oder zu Glycosylaminen mit intakter Ringstruktur umzusetzen (Fig. 2.2).

Für die Kopplung der Amino-modifizierten Polysaccharide an die verschiedenen Seitenkettenfunktionen des Proteins bietet sich der Einsatz eines bifunktionellen Linkers an (siehe unten).

### Einführung von Amino-Funktionen durch reduktive Aminierung

Im Gegensatz zu der Glycaminsynthese durch reduktive Aminierung mit NH₃ oder aliphatischen Aminen (B. Kuberan et al., Glycoconj. J. 1999, 16, 271-81), können mit aromatischen Aminen wie z.B. Benzylamin (T. Yoshide, Methods Enzymol. 1994, 247, 55-64), 2-(4-Aminophenyl)ethylamin (APEA) (H.D. Grimmecke, H. Brade, Glycoconj. J. 1998, 15, 555-62) oder 4-Trifluoracetamidoanilin (E. Kallin, Methods Enzymol. 1994, 247, 119-23) unter vergleichbaren Bedingungen höhere Ausbeuten erzielt werden (Fig. 2.2a).

Während bei APEA die unterschiedliche Reaktivität der aliphatischen und aromatischen Aminofunktionen für eine selektive Reaktion ausgenutzt wird, steht mit 4-Trifluoracetamidoanilin eine mono-geschützte Verbindung zur Verfügung (alternativ wird auch Benzyloxycarbonylaminoanilin eingesetzt (M. Barström et al., Carbohydr. Res. 2000, 328, 525-31)), wobei durch nachfolgende Abspaltung der Trifluoracetyl-Gruppe wiederum eine aromatische Aminofunktion freigesetzt wird. Zudem hat sich gezeigt, dass die Glycamine durch eine einfache N-Acetylierung mit Essigsäurenanhydrid vor der Abspaltung der TFA-Schutzgruppe stabilisiert werden können.

### Einführung von Amino-Funktionen durch N-Glycosilierung

Die N-Glycosilierung (Fig. 2.2b) bietet eine Möglichkeit, die cyclische Ringstruktur des reduzierenden Zuckers zu erhalten. Die durch Umsetzung mit Ammoniumhydrogencarbonat erhaltenen instabilen β-Glycosylamine (I.D. Manger et al., Biochemistry 1992, 31, 10724-32; I.D. Manger et al., Biochemistry 1992, 31, 10733-40; S.Y.C. Wong et al., Biochem. J. 1993, 296, 817-25, E. Meinjohannes et al., J. Chem. Soc,. Perkin Trans. 1, 1998, 549-60) lassen sich durch nachfolgende Acylierung mit Chloressigsäureanhydrid stabilisieren und durch Aminolyse zu den 1-N-Glycyl-Verbindungen mit freier Amino-Funktionalität umsetzen. Analog kann die N-Glycosilierung mit Allylamin durchgeführt werden und, nach Stabilisierung durch N-Acetylierung, Cysteamin photochemisch an die Doppelbindung addiert werden (D. Ramos et al., Angew. Chem. 2000, 112, 406-8).

### Darstellung von Amino-Funktionen aus den Aldonsäuren

In die durch Oxidation von reduzierenden Polysacchariden zugänglichen Aldonsäuren können durch Umsetzung mit Diaminen freie Aminofunktionen eingeführt werden. Dies ist durch die Reaktion der Säure mit Carbodiimiden und Diaminen möglich. Alternativ können die durch Dehydratisierung der Aldonsäuren zugänglichen Lactone mit Diaminen umgesetzt werden (S. Frie, Diplomarbeit, Fachhochschule Hamburg, 1998).

### Kopplungsreaktionen von modifizierter HES und allergenen Proteinen mittels bifunktioneller Linker

So vielfältig wie die funktionellen Gruppen modifizierter HES und Protein-Seitenketten, die über einen Linker miteinander verbunden werden sollen, sind auch die zur Verfügung stehenden Reaktionsmöglichkeiten (Fig. 3 zeigt gängige Linker-Aktivierungen).

Bei den reaktiven Gruppen kann unterschieden werden zwischen Reaktivität gegenüber Amino-Gruppen (NHS-Ester, Imido-Ester und Arylazide), Aldehyden und (in Gegenwart von EDC) Carbonsäuren (Hydrazide) oder SH-Gruppen (Maleinimide, Halogenacetate oder Pyridyldisulfide).

### Reagenzien mit Amin-Reaktivität

Unter den Kopplungsreagenzien sind die Amin-reaktiven Cross-Linker die gebräuchlichsten. Dabei stellen die N-Hydroxysuccinimid (NHS)-Ester (Fig. 3.1a) die gängigste Form der Aktivierung dar. Unter Abspaltung von NHS werden hierbei die acylierten Verbindungen gebildet. Eine weitere Möglichkeit zur Modifizierung primärer Amine bieten die Imido-Ester (F.C. Hartman, F. Wold, Biochemistry 1967, 6, 2439-48) (Fig. 3.1b), wobei Imidoamide (Amidine) gebildet werden. Die Imidoester werden vielfach als Protein-Crosslinker eingesetzt und zeichnen sich durch minimale Reaktivität gegenüber anderen Nucleophilen aus. Weiterhin stehen verschiedene Arylazid-Linker zur Verfügung (photoreaktive Cross-Linker), bei denen durch Photolyse kurzlebige Nitrene gebildet werden. Über eine Ringerweiterung entstehen hieraus (anstatt einer unspezifischen Insertion) Dehydroazepine, die bevorzugt mit Nucleophilen, insbesondere Aminen, reagieren (Fig. 3.1c).

Aufgrund der Vielzahl kommerziell erhältlicher Kopplungsreagenzien mit Amino-Aktivität und variablen Linkern haben andere Reaktionsmöglichkeiten, wie z.B. die Umsetzung mit Isocyanaten und Isothiocyanaten zunehmend an Bedeutung verloren.

### Reagenzien mit Reaktivität gegenüber Carbonyl- oder Carboxyl-Gruppen

Hydrazid-Linker werden für die Kopplung von Verbindungen mit Carbonyl- oder Carboxy-Gruppen verwendet (D.J. O'Shanessy, M. Wilchek, Anal. Biochem 1990, 191, 1-8) (Fig. 3.2). Während Aldehyde zu Hydrazonen umgesetzt werden, die durch Reduktion mit NaCN/BH₃ stabilisiert werden können, reagieren Carboxyl-Gruppen in Gegenwart von EDC unter Bildung von Imid-Bindungen. Die Hydrazid-aktivierten Linker stellen eine vielseitige Alternative zur reduktiven Aminierung und zu den Carboxyl-Aktivierungen mit 'Zero-length' Cross-Linkern wie Carbonyldiimidazol (CDI) dar.

### Reagenzien mit Sulfhydryl-Reaktivität

Kopplungsreagenzien mit SH-Reaktivität stellen eine zweite große Klasse von Cross-Linkern dar. Die Kopplungsreaktionen umfassen primär zwei Reaktionswege: Alkylierung (Fig. 3.3a-b) oder Disulfid-Austausch (Fig. 3.3c). Unter Ausbildung einer stabilen Thioether-Bindung kann, neben der Alkylierung mit α-Halogenacetaten, die Doppelbindung der Maleinimide durch eine Michael-Addition selektiv mit SH-Gruppen umgesetzt werden. Der Thiol-Disulfid-Austausch stellt eine weitere Sulfhydrylspezifische Reaktion dar. Hierbei erweist sich die Reaktion mit den Pyridyldisulfiden (J. Carlsson et al., Biochem J. 1978, 173, 723-37) als besonders vorteilhaft, da unter Abspaltung von 2-Pyridon ein vollständiger Umsatz zu den gemischten Disulfiden erreicht werden kann.

### Cross-Linker

Für die Synthese der erfindungsgemäßen HAS-Allergen-Biokonjugate kommen die obengenannten Kopplungsreaktionen durch vielfältige homo- und heterobifunktionelle Cross-Linker zur Anwendung.

### Homobifunktionelle Cross-Linker

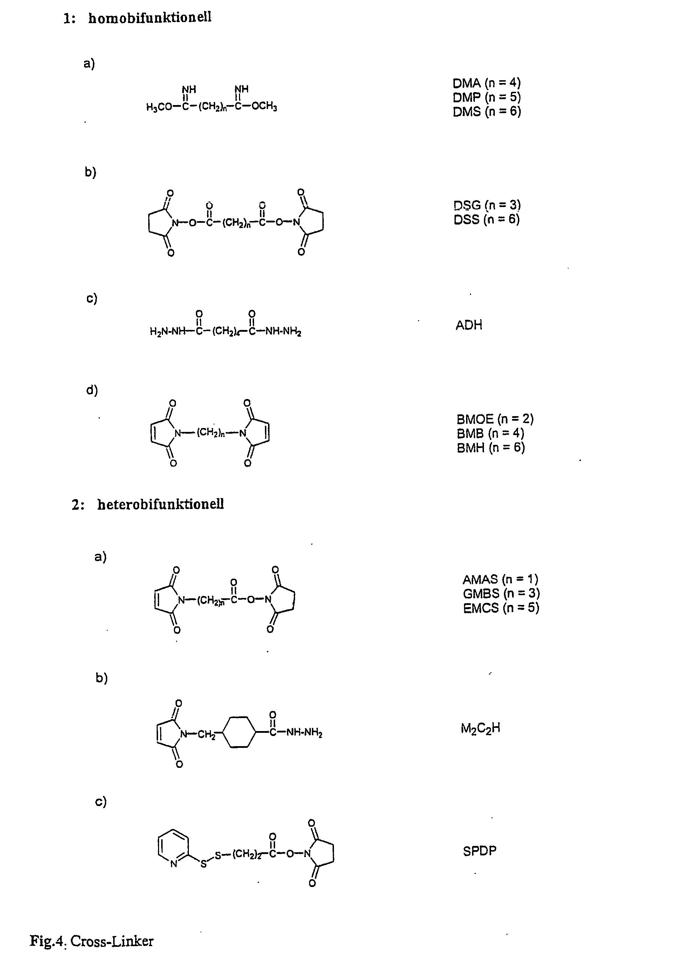

Symmetrische homobifunktionelle Linker (vgl. z.B. die in Fig. 4.1 dargestellten) besitzen an beiden Enden dieselbe reaktive Gruppe und sind dazu geeignet, Verbindungen mit gleichen funktionellen Gruppen miteinander zu verknüpfen. Gemäß den zur Verfügung stehenden Kopplungsreaktionen sind entsprechende bifunktionelle Linker mit z.B. Bis-Imidoester, Bis-Succinimid, Bis-Hydrazid und Bis-Maleinimid-Funktionalitäten kommerziell erhältlich.

Ein Nachteil in der Verwendung von homobifunktionellen Linkern besteht darin, dass selbst bei der Verwendung eines großen Überschusses an Cross-Linker bei der Aktivierung der ersten Verbindung ein Cross-Linking nicht vollständig verhindert werden kann (S. Bystrick et al., Glycoconj. J. 1999, 16, 691-95). Dessen vollständige Abtrennung ist vor der Kopplung mit der zweiten Verbindung notwendig und kann bei Instabilität des aktivierten Zwischenproduktes schwierig sein (z.B. Hydrolyseempfindlichkeit der NHS-aktivierten Verbindungen). Sowohl Amin-Reaktivität als auch die Hydrolyse der NHS-Ester nehmen mit steigendem pH-Wert zu, weshalb Reaktionen unter physiologischen Bedingungen (pH 7) in gepufferten Lösungen durchgeführt werden (Halbwertszeit des NHS-Esters DSP beträgt bei 0°C und einem pH-Wert von 7, 4-5 Stunden, bei pH 8.6 nur noch 10 min; A.J. Lomant, G. Fairbanks, J. Mol. Biol. 1976, 104, 243-261).

### Heterobifunktionelle Cross-Linker

Mit heterobifunktionellen Kopplungsreagenzien (vgl. z.B. die Fig. 4.2 dargestellten) lassen sich Verbindungen mit unterschiedlichen funktionellen Gruppen miteinander verknüpfen. Die Linker sind mit zwei verschiedenen reaktiven Gruppen versehen und durch Kombination verschiedener Kopplungsreaktionen kann selektiv an einem Ende des Cross-Linkers umgesetzt werden. So zeigt z.B. die eine Seite des Linkers Amino-, die andere Sulfhydryl-Aktivität, wodurch sich gegenüber den homobifunktionellen Linkern eine bessere Möglichkeit zur Reaktionskontrolle ergibt.

Zunächst wird die reaktivere bzw. instabilere Seite des heterobifunktionellen Linkers umgesetzt. Da NHS-Ester nicht nur mit Amino-Gruppen unter Ausbildung einer stabilen Amidbindung, sondern auch mit Sulfhydryl- und Hydroxyl-Gruppen reagieren können, wird zunächst der heterobifunktionelle Linker mit der Amino-Verbidung umgesetzt. Die Maleinimido-Gruppe zeigt demgegenüber nicht nur größere Selektivität, sondern in wässriger Lösung auch eine größere Stabilität, so dass das aktivierte Intermediat aufgereinigt und anschließend selektiv mit der Verbindung mit Sulfhydryl-Aktivität umgesetzt werden kann.

Die Wahl des Cross-Linkers richtet sich nicht nur nach der Art der funktionellen Gruppen, die für die Kopplung genutzt werden sollen, sondern auch nach gewünschter Länge und Zusammensetzung, der sogenannten 'cross-bridge' des Spacers. So rufen einige Spacer, insbesondere solche mit starrer Ringstruktur wie z.B. SMCC oder MBS, eine spezifische Antikörper-Reaktion hervor (J.M. Peeters et al., J. Immunol. Methods 1989, 120, 133-43) und können somit für Hapten-Carrier-Immunogene und eine *in vivo* Anwendung weniger geeignet sein.

Bei der Zusammenstellung der Linker in Fig. 4 sind die spezifisch spaltbaren Linker, die sich durch Disulfid-Spaltung (z.B., DSP, DTME oder DTBP) oder Periodat-Spaltung (Diole wie BMDB oder DST) öffnen lassen und die zur Studie biospezifischer Interaktionen oder zur Reinigung unbekannter Target-Strukturen verwendet werden, ausgelassen worden.

Die verwendeten Abkürzungen der kommerziell erhältlichen Kopplungsreagenzien sind aus den systematischen Namen der Verbindungen abgeleitet, wie z.B. DMA (Dimethyladipimidat), DMS (Dimethylsuberimidat), GMBS (*N*-(γ-Maleimidobutyryloxy)succinimidester) etc.

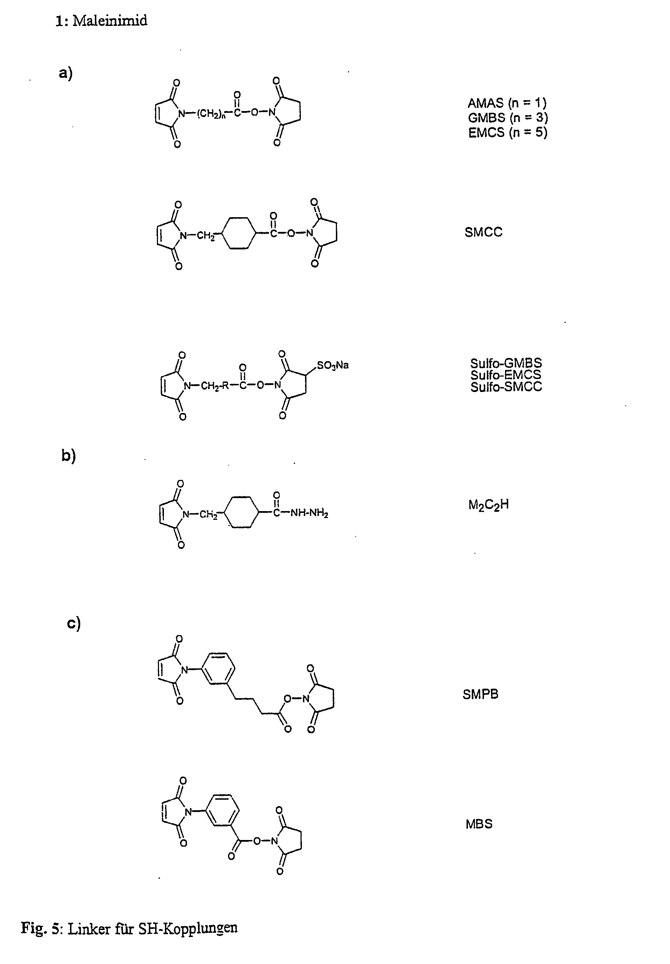

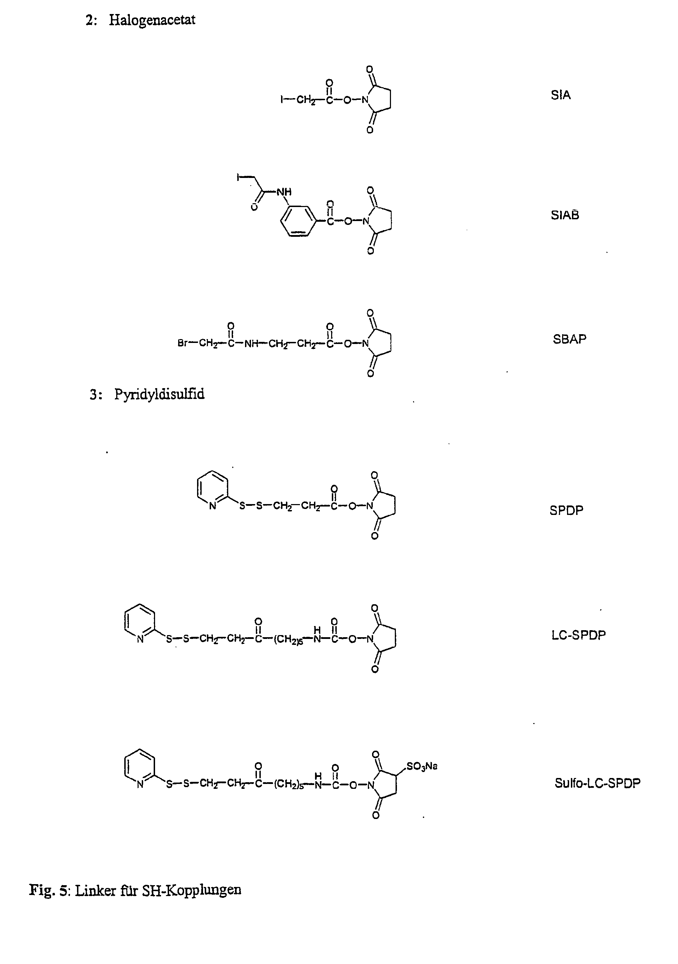

Eine Übersicht über verschiedene heterobifunktionelle Cross-Linker, die beispielsweise für Sulfhydryl-Kopplungen genutzt werden könnten, ist in Fig. 5 gezeigt.

Die größte Vielfalt bieten hier die Maleinimid-aktivierten Linker, meist kombiniert mit einer NHS-Ester-Aktivierung. Diese Linker mit Sulfhydryl- und Amino-Reaktivität sind wasserunlösliche, linear Alkyl-verbrückte Linker wie z.B. AMAS, GMBS und EMCS oder besitzen wie SMCC, SMPB oder MBS eine starre Ringstruktur. Die beiden UV-aktiven Linker SMPB und MBS werden üblicherweise für immunchemische Verfahren wie ELISA-Tests verwendet.

Mit M₂C₂H ist darüberhinaus ein Linker mit derselben starren Verbrückung wie bei SMCC, aber mit Hydrazid-Aktivierung zur Verknüpfung von Verbindungen mit Sulfhydryl- und Carbonyl- oder Carboxyl-Aktivität gegeben.

Im Gegensatz zu den wasserunlöslichen Linkern, die vor der Reaktion zunächst in einem organischen Lösungsmittel wie DMF oder DMSO gelöst werden müssen, stehen mit den hydrophylen Sulfo-NHS Estern (J.V. Staros, Biochemistry 1982, 21, 3950-55), wie z.B. Sulfo-GMBS, Sulfo-EMCS und Sulfo-SMCC, außerdem die wasserlöslichen Varianten einiger Linker zur Verfügung.

Neben den Maleinimid-aktivierten heterobifunktionellen Linkern sind für Sulfhydryl-Kopplungen ferner diverse Halogenacetate, wie z.B. SIA (und das Brom-Analogon), SIAB und SBAP (Fig. 5.2), und Pyridyldisulfide wie SPDP und LC-SPDP und SUlfo-LC-SPDP (Fig. 5.3), wiederum in Kombination mit einer NHS-Ester Aktivierung für eine Amino-Kopplung, verwendbar. Halogenacetate können in aminierte Polysaccaride auch durch eine Reaktion mit der freien Säure und wasserlöslichem Carbodiimid (N.J. Davies, S.L. Flitsch, Tetrahedron Lett. 1991, 32, 6793-6796) bzw. mit dem entsprechenden Anhydrid (I.D. Manger et al., Biochemistry 1992, 31, 10733-40; S.Y.C. Wong et al., Biochem. J. 1994, 300, 843-850) eingeführt werden (vgl. Fig. 2.2b).

Für die Kopplung synthetischer Oligosaccharide an SH-Seitenketten von Proteinen über heterobifunktionelle Maleinimid-Linker sind in der Literatur diverse Beispiele zu finden (V. Fernandez-Santana et al., Glycoconj. J. 1998, 15, 549-53; G. Ragupathi et al., Glycoconj. J. 1998, 15, 217-21; W. Zou et al., Glycoconj. J. 1999, 16, 507-15; R. Gonzalez-Lio, J. Thiem, Carbohydr. Res. 1999, 317, 180-90). Außerdem werden auch direkte Kopplungen von Iodacetatamid-Derivaten von Oligosacchariden für die spezifische Glycosylierung von Proteinen verwendet (N.J. Davies, S.L. Flitsch, Tetrahedron Lett. 1991, 32, 6793-679645; S.Y.C. Wong et al., Biochem. J. 1994, 300, 843-850).

### Modifikation von Glycoproteinen am Glyco-Teil mit Poly- und Oligosacchariden:

Alternativ zu den Aminosäure-Seitenketten des Proteins bieten bei Glycoproteinen auch die gebundenen Oligosaccharide weitere Verknüpfungsstellen zur Bildung der erfindungsgemäßen Konjugate (J.J. Zara et al., Anal. Biochem. 1991, 194, 156-62).

### Einführung von Aldehyden durch eine Oxidation mit Natriumperjodat

Durch die Oxidation mit Natriumperiodat lassen sich auch in nicht reduzierende Oligosaccharide Aldehyde einführen. Je nach Wahl der Oxidationsbedingungen können vorhandene Sialinsäuren selektiv oxidiert werden oder weniger selektiv auch Fucose-, Mannose-, Galactose- und N-Acetyl-Glucosamin-Reste oxidiert werden (S.M. Chamov et al., J. Biol. Chem. 1992, 267, 15916-22). Als Nebenreaktion ist die Bildung von Aldehyden aus n-terminalem Serin, Cystein oder Threonin möglich (D.J. O'Shanessy, M. Wilchek, Anal. Biochem. 1990, 191, 1-8).

### Enzymatische Einführung von Aldehyden

Die Oxidation von Glycoproteinen mit Galactose-Oxidase führt zur Bildung von C6-Aldehyden an terminalen Galactosen oder N-Acetylgalactosaminen. Besonders in Glycoproteinen aus tierischen Zellen sind diese Zucker jedoch nicht terminal, sodass sie :erst in einem vorangehenden Schritt zugänglich gemacht werden müssen (D.J. O'Shanessy, M. Wilchek, Anal. Biochem. 1990, 191, 1-8).

### PHARMAZEUTISCHE ZUSAMMENSETZUNGEN

Die vorliegende Erfindung betrifft die Verwendung eines HAS-Allergen-Konjugats zur Herstellung eines Arzneimittels zur Hyposensibilisierung. Die beschriebenen Konjugate sind in besonders vorteilhafter Weise zur Herstellung von pharmazeutischen Zusammensetzungen geeignet, welche für die Hyposensibilisierung von Allergikern eingesetzt werden können. Die pharmazeutischen Zusammensetzungen sind insbesondere zur Therapie von Allergikern geeignet, bei denen eine IgE-vermittelte Sensibilisierung nachgewiesen wurde und entsprechende klinische Symptome beobachtet wurde.

Demgemäß können die beschriebenen Konjugate insbesondere zur Herstellung von pharmazeutischen Zusammensetzungen verwendet werden, welche zur spezifischen Immuntherapie von Patienten mit klinisch relevanten Reaktionen auf Sofort-Typ-Allergene geeignet sind, wie beispielsweise Pollen-, Milben-, Säugetierhaar(-speichel)-, Pilz-, Insekten-, Nahrungsmittel- und Naturkautschuk/Latex-Allergiker. Die Immuntherapie eignet sich somit insbesondere für die Behandlung von Asthmatikern und Heuschnupfen-Patienten.

Die Zusammensetzungen können in verschiedenen Formen zur Hyposensibilisierung eingesetzt werden. So kann die Hyposensibilisierung durch subkutane, mukosale, orale, perorale oder sublinguale Verabreichung der erfindungsgemäßen HES-Konjugate erfolgen. Ferner kann die Hyposensibilisierung in Form verschiedener Behandlungsprotokolle (präsaisonal/perennial) durchgeführt werden.

Insbesondere bei Insektenallergikern kann es sich anbieten, die Therapie nach dem Rush- oder Ultra-Rush-Verfahren durchzuführen (vgl. Kleine-Tebbe et al., Pneumologie, Vol. 5 (2001), 438-444).

Für die Herstellung der pharmazeutischen Zusammensetzungen werden die Konjugate mit für die Hyposensibilisierung geeigneten Träger- und/oder Hilfsstoffen vermischt.

### Konjugat aus HES und allergenem Glykoprotein

Bei der Herstellung von HES-Glykoprotein-Konjugaten können beispielsweise die folgenden Typen chemischer Funktionalitäten des Glykoproteins für die Kopplung genutzt weden:
A: die Thiol-Gruppe einer Cystein-Seitenkette
B: die Aldehydgruppe eines oxidierten Galactose-Restes.

Bei Proteinen, die nicht glykolysiert sind, entfällt demgemäß Alternative B.

HES zeichnet sich durch ein einziges reduktives Ende aus. Aufgrund dieses Strukturmerkmals ist HES im Rahmen der vorliegenden Erfindung besonderes gut für eine gezielte regioselektive Verknüpfung geeignet.

Für die HES-Protein-Konjugat-Synthese können Konzepte der chemischen Ligation zum Einsatz gelangen, welche für die Konstruktion von größeren Proteinen aus ungeschützten Peptidfragmenten entwickelt wurden. Diese Konzepte basieren auf der Wahl von j e-weils singulären reaktionsfähigen Funktionen in den zu verknüpfenden Fragmenten, die in Gegenwart der Vielzahl anderer Funktionen in natürlichen Proteinen selektiv miteinander zu einem stabilen Endprodukt reagieren.

Im Allgemeinen wird das HES-Präparat zunächst in ein definiertes, hochgereinigtes und gut charakterisiertes Zwischenprodukt (reactivHES) überführt, welches dann spontan und regioselektiv unter physiologischen Bedingungen mit der Zielfunktion des Allergens reagieren kann.

Bevorzugt wird die selektive Überführung des reduktiven Endes von HES in eine primäre Aminofunktion (1-Amino-HES). Dieses »1-Amino-HES« kann dann in flexibler Weise an die Verknüpfungsreaktion mit dem Protein angepasst werden, wobei verschiedene Synthesewege durchlaufen werden können und Reaktionsschritte durch vorgefertigte Reagenzien (Linker) zu einem Schritt vereinigt werden können.

### HS-reactivHES

Im folgenden werden alternative Verfahren zur Herstellung von HS-reactivHES schematisch beschrieben und bewertet:
1.
   - Reduktive Aminierung von HES mit dem bifunktionellen Linker M₂C₂H (Fig. 5.1.b) zum HS-reactivHES (**A**);
   - Reinigung durch Dialyse und Gefriertrocknung;
   - Kopplung des HS-Proteins durch Michael-Addition.
      Diese Synthese weist besondere Vorteile auf, da sie sehr einfach ist (1-Schritt) und die Reaktion mit dem Zielprotein erfolgt sehr selektiv. Sofern die Toxizität von Hydrazinderivaten Probleme bereitet, müssen diese anschließend durch im Stand der Technik bekannte Reinigungsverfahren aufgereinigt werden.
2.
   - Umsetzen des HES-Lactons (oxidiertes HES) mit dem bifunktionellen Linker M₂C₂H (Fig. 5.1.b) zum HS-reactivHES (**B**);
   - Reinigung durch Dialyse und Gefriertrocknung;
   - Kopplung des HS-Proteins durch Michael-Addition.
      Diese Reaktion unterscheidet sich von der oben unter 1. Beschriebenen durch zusätzlichen Aufwand für die Herstellung des HES-Lactons.
3.
   - Umsetzung von HES mit Ammoniumhydrogencarbonat zu 1-Amino-HES (**C**);
   - Reinigung durch Gefriertrocknung;
   - Acylierung des 1-Aminals mit Brom/Jodacetanhydrid ohne Basenkatalyse zum Brom/Jodacetamid (HS-reactivHES **D**);
   - Reinigung durch Dialyse und Gefriertrocknung; Kopplung mit HS-Protein durch Alkylierung.

   Auch dieses Verfahren ist vorteilhaft; es umfaßt nur zwei Schritte und braucht nur sehr einfache Reagenzien. Das Verfahren ist somit sehr kostengünstig. Der Synthesemaßstab ist leicht erweiterbar. Die Reaktion mit dem Zielprotein ist sehr selektiv.
4.
   - Umsetzen des HES-Lactons (oxidiertes HES) mit einem Diamin (1,4-Diaminobutan) nach Frie (S. Frie, Diplomarbeit, Fachhochschule Hamburg, 1998) zu einem Amino-HES (**E**);
   - Acylierung des Amino-HES mit Brom/Jodacetanhydrid ohne Basenkatalyse zum Brom/Jodacetamid (HS-reactivHES **F**);
   - Reinigung durch Dialyse und Gefriertrocknung; Kopplung mit HS-Protein durch Alkylierung.

   Dieser Syntheseweg unterscheidet sich von dem oben unter 3. Beschriebenen durch zusätzlichen Aufwand für die Herstellung des HES-Lactons.

### CHO-reactivHES

Im folgenden werden alternative Verfahren zur Herstellung von CHO-reactivHES schematisch beschrieben und bewertet:
1.
   - Verwendung des Amino-HES (**E**) als CHO-reactivHES **G**;
   - Kopplung mit CHO-Protein durch reduktive Aminierung.
      Diese Synthese ist sehr einfach und kostengünstig. Die Konkurrenz interner Lysine kann gegebenenfalls Probleme erzeugen, die durch die Wahl der Reaktionsbedingungen zu kontrollieren sind.
2.
   - Umsetzung des HAS-Lactons (oxidiertes HES) mit Hydrazin zum Hydrazid (CHO-reactivHES **H**);
   - Reinigung durch Dialyse und Gefriertrocknung;
   - Kopplung mit CHO-Protein durch Hydrazonbildung bei pH 5-6; vorzugsweise sollte die Kopplungsreaktion gleich *in situ* während der oxidativen Bildung (enzymatisch oder chemisch) aus Galaktose-Resten durchgeführt werden; optional wird eine anschließende reduktive Stabilisierung mit NaCN/BH₃ durchgeführt;
   - eine enzymatische Oxidation der Galactosen sollte bevorzugt mit einem polymergebundenen Enzym durchgeführt werden, um die Abreinigung des Enzyms zu erleichtern.

   Diese Synthese ist sehr einfach und selektiv (keine Konkurrenz interner Lysine). Probleme könnten sich aufgrund der Toxizität der Hydrazinderivate ergeben.
3.
   - Weitere Umsetzung von **D** oder **F** mit Ammoniumhydrogencarbonat zu den Glycinamiden (CHO-reactivHES **H** und **I**);
   - Reinigung durch Dialyse und Gefriertrocknung;
   - Kopplung mit CHO-Protein durch reduktive Aminierung.
      Dieses Verfahren erfolgt in drei Schritten, braucht jedoch sehr einfache Reagenzien und ist somit kostengünstig. Der Synthesemaßstab ist leicht erweiterbar. Es könnte jedoch zur Konkurrenz interner Lysine kommen (vgl. oben)
4.
   - Acylierung des Amino-HES **C** oder **E** mit cBz-Aminooxyessigsäure mit nachfolgender Hydrierung zum Aminooxy-HES (CHO-reactivHES **K**);
   - Kopplung mit CHO-Protein durch Oxim-Bindung bei pH 5-6; vorzugsweise sollte die Kopplungsreaktion gleich *in situ* während der oxidativen Bildung (enzymatisch oder chemisch) aus Galaktose-Resten erfolgen;
   - eine enzymatische Oxidation der Galactosen sollte bevorzugt mit einem polymergebundenen Enzym durchgeführt werden, um die Abreinigung des Enzyms zu erleichtern.

   Diese Synthese ist aufwendig, aber die Kopplung mit dem Zielprotein ist ebenso selektiv wie bei der unter 2. beschriebenen Reaktion (keine Konkurrenz interner Lysine).

## Patentansprüche

1. Verwendung eines Konjugates aus Hydroxyalkylstärke und einem Allergen, bei dem mindestens eine Hydroxyalkylstärke kovalent an das Allergen gekoppelt ist, zur Herstellung eines Arzneimittels zur Hyposensibilisierung.

2. Verwendung gemäß Anspruch 1, wobei die Hydroxyalkylstärke unmittelbar oder über einen Linker an das Allergen gekoppelt vorliegt.

3. Verwendung gemäß Anspruch 1 oder 2, wobei die Hydroxyalkylstärke Hydroxyethylstärke, Hydroxypropylstärke oder Hydroxybutylstärke ist.

4. Verwendung gemäß einem der Ansprüche 1 bis 3, bei der die Hydroxyethylstärke ein mittleres Molekulargewicht von 1 bis 300 kDa, vorzugsweise ein mittleres Molekulargewicht von 5 bis 200 kDa aufweist.

5. Verwendung gemäß einem der vorstehenden Ansprüche, bei der die Hydroxyethylstärke einen molaren Substitutionsgrad von 0,1 bis 0,8 und ein Verhältnis von C₂:C₆ Substitution im Bereich von 2 bis 20, jeweils bezogen auf die Hydroxyethylgruppen, aufweist.

6. Verwendung gemäß einem der vorstehenden Ansprüche, bei der das Allergen aus der Gruppe bestehend aus Polypeptiden oder Proteinen ausgewählt wurde.

7. Verwendung gemäß einem der vorstehenden Ansprüche, bei der das Allergen ein Glykoprotein ist.

8. Verwendung gemäß einem der vorstehenden Ansprüche, bei der die Hydroxyalkylstärke an die Polypeptidkette oder an eine oder mehrere der Saccharidketten des Glykoproteins gekoppelt ist.

9. Verwendung gemäß einem der vorstehenden Ansprüche zur Hyposensibilisierung von Allergikern, bei denen eine IgE-vermittelte Sensibilisierung nachgewiesen ist oder deren klinische Symptome beobachtet wurden.

10. Verwendung gemäß einem der vorstehenden Ansprüche, wobei die Hyposensibilisierung zur Therapie von Pollen-, Milben-, Säugetierhaar(-speichel)-, Pilz-, Insekten-, Nahrungsmittel- und Naturkautschuk/Latex-Allergien eingesetzt wird.

11. Verwendung gemäß einem der vorstehenden Ansprüche, wobei die Therapie für die Behandlung von Asthmatikern, Heuschnupfen-Patienten und Patienten, die anderweitige klinisch relevante Reaktionen auf Soforttyp-Allergene aufweisen, eingesetzt wird.

12. Verwendung gemäß einem der vorstehenden Ansprüche, wobei die Verabreichung subkutan, mukosal, oral, peroral oder sublingual erfolgt.

13. Verwendung gemäß einem der vorstehenden Ansprüche, wobei die Immuntherapie bei Aeroallergenen präsaisonal oder perennial durchgeführt wird.

14. Verwendung gemäß einem der vorstehenden Ansprüche, wobei die Immuntherapie bei Insektenallergikern im Rush- oder Ultra-Rush-Verfahren durchgeführt wird.

## Claims

1. Use of a conjugate of hydroxyalkylstarch and an allergen, wherein at least one hydroxyalkylstarch is covalently coupled to the allergen, for the manufacture of a medicament for hyposensitization.

2. The use according to claim 1, wherein the hydroxyalkylstarch is coupled directly or via a linker to the allergen.

3. The use according to claim 1 or 2, wherein the hydroxyalkylstarch is hydroxyethylstarch, hydroxypropylstarch or hydroxybutylstarch.

4. The use according to any of claims 1 to 3, wherein the hydroxyethylstarch has an average molecular weight of from 1 to 300 kDa, preferably an average molecular weight of from 5 to 200 kDa.

5. The use according to any of the preceding claims, wherein the hydroxyethylstarch has a molar degree of substitution of from 0.1 to 0.8 and a C₂:C₆ substitution ratio in the range of from 2 to 20, in each case based on the hydroxyethyl groups.

6. The use according to any of the preceding claims, wherein the allergen has been selected from the group consisting of polypeptides or proteins.

7. The use according to any of the preceding claims, wherein the allergen is a glycoprotein.

8. The use according to any of the preceding claims, wherein the hydroxyalkylstarch is coupled to the polypeptide chain or to one or more of the saccharide chains of the glycoprotein.

9. The use according to any of the preceding claims for hyposensitization of allergy sufferers in whom an IgE-mediated sensitization is detected or the clinical symptoms of which have been observed.

10. The use according to any of the preceding claims, wherein the hyposensitization is employed for the therapy of allergies to pollen, mites, mammalian hair (- saliva), fungi, insects, foods and natural rubber/latex.

11. The use according to any of the preceding claims, wherein the therapy is employed for the treatment of asthmatics, hay-fever patients and patients showing other types of clinically relevant reactions to immediate-type allergens.

12. The use according to any of the preceding claims, wherein administration takes place subcutaneously, mucosally, orally, perorally or sublingually.

13. The use according to any of the preceding claims, wherein the immunotherapy is carried out preseasonally or perennially for airborne allergens.

14. The use according to any of the preceding claims, wherein the immunotherapy is carried out for people allergic to insects in the rush or ultra-rush method.

## Revendications

1. Utilisation d'un conjugué d'hydroxyalkylamidon et d'un allergène, dans lequel au moins un hydroxyalkylamidon est lié de façon covalente par covalence à l'allergène, pour la préparation d'un médicament destiné à l'hyposensibilisation.

2. Utilisation selon la revendication 1, dans laquelle l'hydroxyalkylamidon est couplé à l'allergène directement ou par l'intermédiaire d'un lieur.

3. Utilisation selon les revendications 1 ou 2, dans laquelle l'hydroxyalkylamidon est un hydroxyéthylamidon, un hydroxypropylamidon ou un hydroxybutylamidon.

4. Utilisation selon les revendications 1 à 3, dans laquelle l'hydroxyéthylamidon a un poids moléculaire moyen compris entre 1 et 300 kDa et de préférence un poids moléculaire moyen compris entre 5 et 200 kDa.

5. Utilisation selon l'une des revendications précédentes, dans laquelle l'hydroxyéthylamidon présente un degré de substitution molaire de 0,1 à 0,8 et un rapport entre les substituants en C₂ et en C₆ compris dans la plage de 2 à 20, chaque fois par rapport aux groupes hydroxyéthyle.

6. Utilisation selon l'une des revendications précédentes, dans laquelle l'allergène est sélectionné dans l'ensemble constitué des polypeptides et des protéines.

7. Utilisation selon l'une des revendications précédentes, dans laquelle l'allergène est une glycoprotéine.

8. Utilisation selon l'une des revendications précédentes, dans laquelle l'hydroxyalkylamidon est accouplé à la chaîne de polypeptide ou à une ou plusieurs des chaînes de saccharide de la glycoprotéine.

9. Utilisation selon l'une des revendications précédentes, pour l'hyposensibilisation à des allergènes, dans laquelle on détecte la sensibilisation conférée par IgE ou dont on observe les symptômes cliniques.

10. Utilisation selon l'une des revendications précédentes, dans laquelle l'hyposensibilisation est utilisée pour la thérapie d'allergies provoquées par le pollen, les acariens, les poils (la salive) de mammifères, les champignons, les insectes, les aliments, le caoutchouc naturel et/ou le latex.

11. Utilisation selon l'une des revendications précédentes, dans laquelle la thérapie est mise en oeuvre pour le traitement de patients souffrant d'asthme, de patients souffrant de rhume des foins et de patients qui présentent des réactions cliniques d'une autre nature à des allergènes de type immédiat.

12. Utilisation selon l'une des revendications précédentes, dans laquelle l'administration s'effectue par voie sous-cutanée, mucosale, orale, perorale ou sublinguale.

13. Utilisation selon l'une des revendications précédentes, dans laquelle l'immunothérapie vis-à-vis d'aéroallergènes est réalisée de manière pré-saisonnière ou pérenne.

14. Utilisation selon l'une des revendications précédentes, dans laquelle l'immunothérapie vis-à-vis des insectes allergènes est menée dans un procédé dans des conditions d'urgence ou d'ultra-urgence.
